# EUROPÄISCHE PATENTSCHRIFT

(11) **EP 0 613 386 B1**
(45) Veröffentlichungstag und Bekanntmachung des Hinweises auf die Patenterteilung: **14.02.1996**
(21) Anmeldenummer: 92923276.7
(22) Anmeldetag: 12.11.1992
(51) Int. Cl.: A61M 25/01, A61B 17/28

(54) **SONDE FÜR MEDIZINISCHE EINGRIFFE IN KÖRPERHÖHLEN**
MEDICAL PROBE FOR INSERTION IN THE CAVITIES OF THE BODY
SONDE POUR INTERVENTIONS MEDICALES DANS DES CAVITES DU CORPS

(30) Priorität: 18.11.1991 DE 4137950
(43) Veröffentlichungstag der Anmeldung: 07.09.1994
(73) Patentinhaber: FRIMBERGER, Eckart, D-80804 München (DE)
(72) Erfinder: PFLUGBEIL, Peter, D-8000 München 83 (DE)
(74) Vertreter: Körber, Wolfhart, Dr. rer.nat.
(86) Internationale Anmeldenummer: EP9202606
(87) Internationale Veröffentlichungsnummer: WO9309835

(56) Entgegenhaltungen:
- DE-U- 8 632 015
- US-A- 3 547 103
- US-A- 4 215 703

## Beschreibung

Die Erfindung bezieht sich auf eine Sonde nach dem Oberbegriff des Anspruchs 1.

Sonden dieser Art werden in verschiedenen Bereichen der Medizin eingesetzt, nämlich zur therapeutischen oder diagnostischen Zwecken in Körperhöhlen des zu behandelnden Körpers, insbesondere zur Einführung in den Verdauungs- oder Ausscheidungstrakt.

Einer solchen Sonde liegt eine Hauptfunktion zugrunde, nämlich eine Relativverschiebung zwischen dem Mantel und der Seele zwecks Ausübung einer spezifischen Funktion oder Manipulation in der Körperhöhle.

Bei einer Sonde mit einer Sklerosierungskanüle wird eine nach vorne gerichtete Ausschubbewegung der Seele zum Injizieren ausgenutzt. Hierzu wird die Seele manuell im Mantel vorgeschoben.

Bei einer sogenannten steuerbaren Führungssonde mit abbiegbarer Sondenspitze, die sich insbesondere für die Aufsuche und die Einführung in seitlich weitergehende Körperhöhlen eignet, ist am hinteren Ende der Sonde eine zwischen dem Mantel und der Seele wirksame Antriebsvorrichtung vorgesehen, mittels der eine nach hinten gerichtete Zugkraft und Verschiebung manuell an der Seele ausgeübt werden kann, um die Abbiegung an der Sondenspitze herbeizuführen. Zur Rückführung der Sondenspitze wird die Seele mittels der Antriebsvorrichtung manuell vorgeschoben.

Eine mit der vorbeschriebenen prinzipiell vergleichbare Ausgestaltung ist auch an einer sogenannten Biopsie-Sonde vorhanden, bei der mittels der Antriebsvorrichtung durch manuelles Vorschieben der Seele die Biopsiezange geöffnet wird und durch manuelles Ziehen der Seele nach hinten die Biopsiezange geschlossen wird.

Bei sogenannten Versteifungssonden wird durch die von einer am hinteren Ende der Sonde angeordneten Spannvorrichtung manuell erzeugbare Zugbeaufschlagung der Seele nach hinten eine axiale Kompression des aus hintereinander liegenden Elementen bestehenden Mantels erreicht. Hierbei übt die Seele in vorderem Bereich des Mantels eine Zugspannung auf ihn aus, während der Mantel im hinteren Bereich durch die separate Spannvorrichtung abgestützt ist.

In vielen Fällen wird die Sonde in ihrem in die Körperhöhle eingeschobenen Zustand als Leitelement bzw. Leitschiene für eine Vielzahl von medizinischen Instrumenten benutzt, bspw. Angiographie - Katheter oder Dilatationsinstrumente, die auf der Sonde in das gewünschte Gebiet des Körpers vorgeschoben werden.

Für die Durchführung des jeweiligen Eingriffes wird die Sonde zunächst in die gewünschte Hohlstruktur des Körpers eingeführt, bspw. mittels eines peroral eingeführten Endoskops vom Zwölffingerdarm aus in den Gallengang. Entlang der Sonde wird dann das entsprechende Instrument in das gewünschte Zielgebiet vorgeschoben. Im Gallengang sind dies häufig Hohlsonden (drains), die bei tumorösen Verengungen des Gallengangs (maligne Stenosen) durch die Engsstelle geschoben und hier belassen werden. Sie überbrücken die Stenose und gewährleisten so die Drainage der Galle.

An eine solche Sonde bestehen zwei Hauptanforderungen, nämlich zum einen eine hochgradige Flexibilität für das risikoarme Einführen und Plazieren der Sonde und zum anderen eine bestimmte Rigidität insbesondere für Führungszwecke, wie z. B. eine stabile Führung bei Bougierung, Drain-Plazierung usw. Durch eine gezielte Versteifung der Sonde kann auch die Schwierigkeit einer Schlingenbildung überwunden werden, z. B. im Magen bei dessen Durchquerung.

Eine solche bekannte Sonde, wie sie in der EP-A-0 131 847 beschrieben ist, erfüllt die vorbeschriebenen gegensätzlichen Forderungen ideal. Im entspannten Zustand ermöglicht sie ihre atraumatische Plazierung, und durch Spannen läßt sich ihre gewünschte Rigidität einstellen. Sie besteht aus einem elastisch biegsamen Mantel, in dem eine Seele in ihrer Längsrichtung verschiebbar geführt ist, deren Länge um ein beträchtliches Maß größer bemessen ist, als die Länge des Mantels. Die Seele weist an ihrem vorderen Ende ein kugelförmiges Anschlagteil und am hinteren einen scheibenförmigen Anschlag auf, wobei beide Teile starr mit der Seele verbunden sind. Diese Überlänge der Seele ist erforderlich, um eine Spannvorrichtung zwischen dem Mantel und der Seele ansetzen und die Seele in ihrer Längsrichtung ziehen zu können, um das im vorderen Endbereich vorhandene Anschlagteil der Seele gegen das vordere Ende des Mantels zu ziehen und dadurch die Sonde zu versteifen. Hierzu wird die Spanneinrichtung zwischen dem hinteren Ende des Mantels und dem hinteren scheibenförmigen Anschlag eingesetzt, wonach mit dem beweglichen Spannelement der Spannvorrichtung ein Zug auf die Seele ausgeübt werden kann, was zur Versteifung der Sonde führt.

Eine Sonde der letzteren Art ist auch in der US-A-4 215 703 beschrieben. Bei dieser bekannten Sonde ist die Spannvorrichtung durch zwei Hülsen gebildet, die in dem Bereich, in dem die Seele am hinteren Ende der Sonde den Mantel nach hinten übetragt, koaxial hintereinanderliegend auf der Seele angeordnet sind, und von denen die hintere Hülse relativ zu vorderen Hülse drehbar ist. Dabei sind die einander zugewandten Enden der Hülsen als schräge bzw. schiefe Ebenen ausgebildet, die in der Ausgangsstellung der Spannvorrichtung parallel aneinander anliegen. Durch eine relative Verdrehung der beiden Hülsen zueinander bewirken die schiefen Ebenen deren Streckung, wodurch auf die an der hinteren Hülse befestigte Seele ein Zug ausgeübt wird, der die Versteifung der Sonde herbeiführt.

Aus der US-PS-3,547,103 ist eine entsprechende Sonde bekannt, bei der die zumindest teilweise beabstandeten Wendel des spiralförmig aus einem Draht gewickelten Mantels zur Versteifung der Sonde zusammengezogen werden.

Die bekannten Sonden sind jedoch hinsichtlich ihres baulichen und ihres handhabungs- bzw. bedienungstechnischen Aufwandes nachteilig. Ersteres ist durch das Vorhandensein der Antriebs- oder Spannvorrichtung bedingt, die einen beträchtlichen Bauaufwand darstellt und außerdem der Sonde eine sperrige Bauweise verleiht, die deren Handhabung grundsätzlich erschwert. Letzteres besteht darin, daß insbesondere die Spannvorrichtung - wenn sie nicht noch zusätzlich an die Sonde anzusetzen ist - einen erheblichen manuellen Kraft- und Bedienungsaufwand erfordert, wobei die behandelnde Person eine Betätigung auszuüben hat, die die Behandlung an sich bzw. die Positionierung der Sonde in der jeweils speziellen Einführungsposition behindert. Hierdurch werden die jeweils durchzuführenden Behandlungsmaßnahmen beeinträchtigt sowie die Bedienung und Manipulation erschwert.

Der Erfindung liegt die Aufgabe, bei einer Sonde der eingangs angegebenen Art den Bau- und Bedienungsaufwand zu verringern.

Diese Aufgabe wird durch die Merkmale der Ansprüche 1 oder 13 gelöst. Vorteilhafte Weiterbildungen der Erfindung sind in den abhängigen Unteransprüchen gekennzeichnet.

Bei der erfindungsgemäßen Sonde ist keine zusätzliche Antriebs- oder Spannvorrichtung erforderlich. Zumindest nicht eine solche Antriebs-oder Spannvorrichtung, die sich am hinteren Ende des Mantels abstützt und die Seele aus dem Mantel nach hinten herauszieht. Die Relativverschiebung läßt sich durch ein - oder mehrmaliges Biegen des Mantels herbeiführen. Hierdurch wird sowohl die angestrebte einfachere Bauweise erreicht, weil eine zusätzliche Antriebs- oder Spannvorrichtung in Fortfall kommt, als auch eine einfachere Bedienung erreicht, weil die Sonde, d. h. der Mantel und die Seele, lediglich gebogen zu werden brauchen, was sich handhabungsfreundlich durchführen laßt, ohne daß die behandelnde Person eine erhöhte Aufmerksamkeit aufzubringen hat. Es ist sogar möglich, daß die Biegung der Sonde durch Finger ein und derselben Bedienungshand ausgeführt werden kann, so daß die andere Hand der behandelnden Person anderen Behandlungstätigkeiten gewidmet werden kann und somit Einhandbedienung vorliegt.

Die erfindungsgemäße Lösung beruht unter anderem auf zwei Hauptmerkmalen, nämlich zum einen darauf, daß die Seele in ihrem hinteren Bereich an den Mantel in dessen hinteren Bereich festgelegt ist und daß eine Längsverschiebung zwischen Mantel und Seele durch eine gemeinsame Biegung erreicht wird. Bei einer Biegung der Seele aus ihrer geraden in eine gekrümmte Erstreckung erfolgt eine Kürzung der Seele gegenüber dem Mantel, so daß das vordere Ende der Seele in den Mantel eingezogen wird. Die Seele kann somit Zugkräfte ausüben. Bei einer Biegung der Sonde aus ihrer gekrümmten in ihre gerade Erstreckung führt dagegen die dabei stattfindende Relativverschiebung zu einer Vorbewegung des vorderen Endes der Seele gegenüber dem Mantel. Hierdurch kann die Seele Schubkräfte ausüben.

Die vorgenannte Festlegung zwischen Seele und Mantel braucht nur in eine Richtung wirksam zu sein und sie kann deshalb durch Verschiebungs-Bewegungsbegrenzungsanschläge gebildet sein. In dem Fall, in dem die Seele eine nach hinten gerichtete Zugkraft ausübt, ist im hinteren Bereich der Sonde die Einschubbewegung der Seele gegenüber dem Mantel zu begrenzen. In dem Fall, in dem die Seele eine nach vorne gerichtete Schubkraft ausübt ist im hinteren Bereich der Sonde die Rückbewegung der Seele gegenüber dem Mantel zu begrenzen. Es ist natürlich auch möglich, die Festlegung im hinteren Bereich der Sonde zwischen Seele und Mantel ortsfest zu fixieren, z. B. durch Klemmen, Schweißen, Löten oder Kleben.

In dem Fall, in dem die Zugkraft der Seele dazu ausgenützt wird eine axiale Kompression auf den Mantel auszuüben, ist eine zweite Festlegung zwischen Mantel und Seele im vorderen Bereich der Sonde erforderlich. Auch diese Festlegung braucht nur in eine Richtung wirksam zu sein, weil lediglich ein Einzug der Seele im vorderen Endbereich der Sonde gegenüber dem Mantel begrenzt zu werden braucht, wozu ein Längsverschiebungs-Begrenzungselementenpaar dienen kann. Es ist jedoch auch in diesem Falle möglich, die Seele und den Mantel im vorderen Bereich der Sonde ortsfest zu fixieren, z. B. durch Klemmen, Schweißen, Löten oder Kleben. Die vorgenannte Kompression beim Biegen der Sonde in deren hinteren Bereich führt zur angestrebten Versteifung der Sonde.

Die erfindungsgemäße Lösung beruht somit auf einem völlig anderen Lösungsgedanken als wie es bei der bekannten Sonde der Fall ist. Bei der bekannten Sonde wird der Mantel durch die Antriebsvorrichtung ortsfest gehalten und die Seele wird an ihrem hinteren Ende nach hinten gezogen. Bei der erfindungsgemäßen Lösung findet dagegen durch das Biegen der Sonde eine Relativverschiebung zwischen der Seele und dem Mantel statt, mit der Funktionen und Manipulationen ausgeführt werden können, z. B. Schubkraftausübung oder Zugkraftausübung bzw. Kompression der Mantels.

Da bereits beim Einführen der Sonde in gekrümmte Körperhöhlen eine geringe Längsverschiebung zwischen Mantel und Seele stattfindet, ist insbesondere dann, wenn auch im vorderen Bereich der Sonde eine Festlegung zwischen Mantel und Seele vorgesehen ist, eine Verschiebespiel zwischen Mantel und Seele vorzugeben.

Die Länge des Verschiebespiels ist zum einen durch die Anzahl und Größe von Krümmungen in der Körperhöhle, in die eingegriffen werden soll, und durch die Länge und/oder Stärke der Biegung der Sonde bestimmt. Gegebenenfalls kann die Sonde mehrmals um die Finger oder die Hand der behandelnden Person gewickelt werden. Wesentlich ist bei der Kompression des Mantels, daß durch die Biegung der Sonde das Verschiebespiel aufgehoben wird, wonach aufgrund der Biegung eine axiale Kraft zwischen dem Mantel und der Seele wirksam ist, die zur angestrebten Versteifung führt. In dem Fall, in dem lediglich durch die Biegung der Sonde in deren hinteren Bereich eine Relativverschiebung erreicht werden soll, ist ein Verschiebespiel hinsichtlich beabsichtigter Zielpunkte der angestrebten Bewegung zu berücksichtigen.

Durch ein Zurückbiegen der Sonde, was nach deren seitlichen Ausbiegung aufgrund der Elastizität des Mantels nach dem Löslassen selbsttätig stattfindet, wird die erfindungsgemäß herbeigeführte Bewegung bzw. Aktion wieder zurückgeführt.

Bei der Erfindung wird die angestrebte Bewegung der Seele bzw. das vorbeschriebene Verschiebespiel durch die Festlegung zwischen Mantel und Seele im hinteren Bereich der Sonde begrenzt. Eine solche sondenseitige Festlegung ist bei den bekannten Sonden nicht vorhanden.

Für einen Mantel eignet sich vorzugsweise ein solcher, der durch einen wendelförmig gewickelten elastischen Draht bzw. Federdraht oder ein entsprechendes Band gebildet ist. Dabei ist es im Rahmen der Erfindung möglich, daß mehrere oder alle Wicklungen einen Abstand voneinander aufweisen, wobei die Summe dieser Abstände dem Verschiebespiel entsprechen kann. Bei dieser Ausgestaltung ist aufgrund der federnden Nachgiebigkeit der einzelnen Wicklungen in Längsrichtung zu Beginn der Versteifung eine weichere Kennlinie der Rigidität erreichbar.

Es ist möglich, einen gewickelten Mantel so auszuführen, daß die Wicklungen direkt aneinanderliegen. Bei dieser Ausgestaltung ist das Verschiebespiel durch einen Abstand zwischen dem hinteren Begrenzungselementenpaar gebildet.

Im Rahmen der Erfindung ist es auch möglich, den Mantel aus hintereinander liegenden Gliedern zu bilden, wie es in den Figuren 15 und 16 der vorher erwähnten EP-A-0 131 847 dargestellt und in den zugehörigen Textstellen beschrieben ist. Auf diese Beschreibung wird im vollen Umfang Bezug genommen.

Bei der erfindungsgemäßen Ausgestaltung laßt sich die Versteifungssonde bezüglich ihrer Längsmitte symmetrisch ausgestalten, so daß es ausgestaltungsmäßig keinen Unterschied zwischen ihrem vorderen und ihrem hinteren Ende gibt. Die erfindungsgemäße Sonde kann somit beidseitig verwendet werden.

In den Unteransprüchen sind Merkmale enthalten, die zur Problemlösung beitragen, zu einer einfachen, stabilen und kostengünstig herstellbaren Bauweise führen, die Handhabung weiter erleichtern und die Funktion weiter verbessern.

Nachfolgend werden die Erfindung und weitere durch sie erzielbare Vorteile anhand von bevorzugten Ausführungsbeispielen und einer Zeichnung näher erläutert. Es zeigt
- Figur 1: eine erfindungsgemäß ausgestaltete Sonde in einer nicht versteiften, geraden Neutralstellung und andeutungsweise in einer gebogenen, versteiften Stellung;
- Figur 2: eine erfindungsgemäß ausgestaltete Sonde in einer nicht versteiften, geraden Neutralstellung und andeutungsweise in einer gebogenen, versteiften Stellung als zweites Ausführungsbeispiel;
- Figuren 3 bis 7: erfindungsgemäße Sonden in abgewandelten Ausgestaltungen;
- Figur 8: den Schnitt VIII - VIII in Figur 7;
- Figur 9: eine erfindungsgemäße Sonde mit einer Meßvorrichtung für die Längsverschiebung ihrer Seele oder für die vorhandene Zugspannung;
- Figur 10: die Sonde nach Figur 9 in ihren versteiften Stellung;
- Figur 11: eine erfindungsgemäße Sonde mit einer Biopsiezange an ihrem vorderen Ende und bei geöffneter Biopsiezange;
- Figur 12: die Sonde nach Figur 11 bei geschlossener Biopsiezange;
- Figur 13: eine erfindungsgemäße Sonde mit seitlich abbiegbarer Spitze als sogenannte Führungssonde in zwei Funktionsstellungen;
- Figur 14: eine erfindungsgemäße Sonde mit einer Sklerosierungskanüle an ihrem vorderen Ende in der Einführungsstelle der Sonde;
- Figur 15: die Sonde nach Figur 14 in ihrer Injektionsstellung;
- Figur 16: eine Vorrichtung zum Aufwickeln und Halten des hinteren Endbereichs der Sonde in der Seitenansicht, teilweise längs geschnitten;
- Figur 17: die Vorrichtung nach Figur 16 in der Vorderansicht;
- Figur 18: die Vorrichtung in der Blickrichtung gemäß Pfeil X in Figur 17.

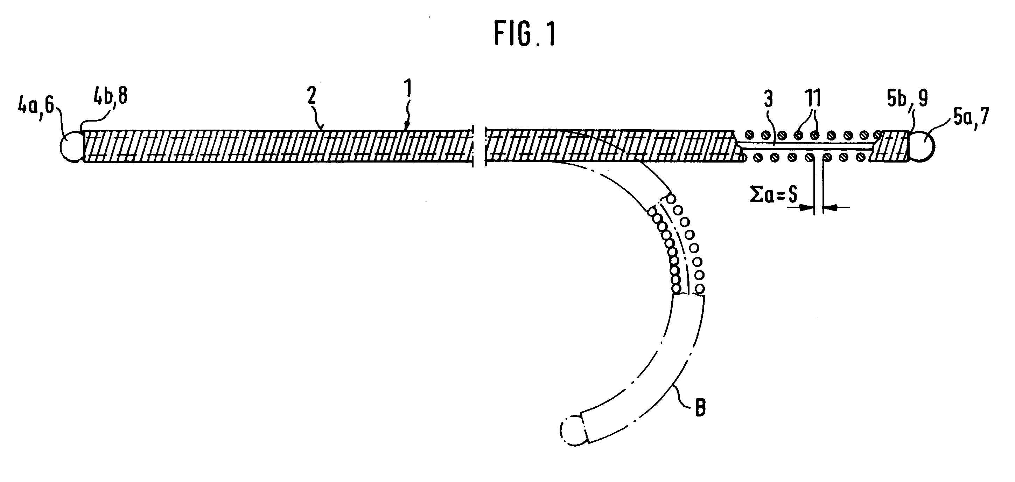

Bei der Sonde 1 gemäß den Figuren 1 bis 10 handelt es sich um eine sogenannte Versteifüngssonde, die ohne Versteifung in die Körperhöhle eines zu behandelnden Körpers einführbar und dann versteifbar ist. Die Sonde 1 besteht aus einem in seiner Umfangsrichtung geschlossenen Mantel 2 und einer darin mit Bewegungsspiel in Längsrichtung verschiebbaren Seele 3, wobei Verschiebungs-Begrenzungselementenpaare 4a, 4b, 5a,5b zwischen dem Mantel 2 und der Seele 3 wirksam sind, die einen axialen Abstand voneinander aufweisen, jeweils im Endbereich der Sonde 1 angeordnet sind und eine Verschiebung des Mantels 2 auf der Seele 3 über ein vorbestimmtes Maß hinaus verhindern.

Die Seele 3 besteht jeweils aus einem biegsamen Draht bzw. Band oder einer biegsamen Litze. Als Material eignet sich Metall, insbesondere Stahl oder Kunststoff vorzüglich. Die Seele ist etwas elastisch, so daß sie nach einer Biegung aus ihrer normalerweise geraden Erstreckung in ihre Ausgangsform leicht zurückfedert. Die miteinander korrespondierenden Begrenzungselementenpaare 4a, 4b, 5a, 5b sind jeweils durch ein am zugehörigen Ende der Seele 3 befestigten verdickten Seelenkopf 6 bzw. 7 und dem zugehörigen Stirnende 8,9 des Mantels 2 gebildet. Die Seelenköpfe 6, 7 sind vorzugsweise Metallköpfe, die unlösbar mit der Seele 3 verbunden sind, z. B. Löten, Schweißen, Kleben oder Pressen. Die Seelenköpfe 6, 7 haben vorzugsweise eine kugelförmige Form und eine Querschnittsgröße, die ein Durchrutschen durch den Mantel 2 verhindert.

Der Mantel 2 besteht aus einem wendelförmig gewickelten elastischen Band oder Draht aus Kunststoff oder Metall, insbesondere Federdraht, wodurch sich in Längsrichtung hintereinander liegende Wicklungen 11 ergeben.

Der freie Innenquerschnitt des Mantels 2 ist so groß bemessen, daß der Draht oder das Band der Seele 3 darin eine radiales Bewegungsspiel hat.

Bei der Ausgestaltung gemäß Figur 1 weisen die einzelnen Wicklungen 11 einen Abstand a voneinander auf, der etwa dem Wicklungsdurchmesser entsprechen kann oder vorzugsweise etwas geringer bemessen ist. Dieser Abstand a kann gezielt gewickelt sein, oder es ist auch möglich, die Abstände a durch Strecken des Mantels 2 über seine Elastizitätsgrenze hinaus zu schaffen. Die Seele 3 ist so lang bemessen, daß deren Seelenköpfe 6, 7 im neutralen, d. h. ungespannten Zustand des Mantels 2 an dessen Stirnenden 8, 9 vorzugsweise anliegen. Die Seelenköpfe können auch einen geringen Abstand von den Stirnenden 8, 9 aufweisen und sie können auch mit einer geringen Spannung an den Stirnenden 8, 9 anliegen. Wenn eine solche Sonde 1 gemäß Figur 1 gebogen wird, nähern sich die Wicklungen 11 an der Innenseite der Biegung und sie gelangen zur gegenseitiger Anlage, wobei bereits jetzt aufgrund der axialen Kraft zwischen den Seelenköpfen 6, 7 und dem Mantel 2 eine Versteifung der Sonde 1 stattfindet. Bei weiterer Biegung B wird die Kraft und Versteifung noch vergrößert. Auf der Außenseite der Biegung B bleiben die Wicklungen 11 voneinander beabstandet.

Die Summe der Abstände a zwischen den Wicklungen 11 ergibt ein Maß, das mit Verschiebespiel 5 zu bezeichnen ist und durch Biegen der Sonde 1 an der Innenseite der Biegung B aufgehoben wird, wie es aus der angedeuteten Darstellung in Figur 1 zu entnehmen ist.

Die Stärke bzw. die Rigidität der Versteifung ist durch die Stärke der Biegung B und/oder die lange der Biegestrecke vorbestimmt. Eine starke Biegung und/oder lange Biegunsstrecke führt zu einer starken Versteifung. Dabei ergibt sich aufgrund der axialen Beabstandung der Wicklungen 11 voneinander eine weiche Kennlinie der Rigidität. Durch Rückbiegung der Biegung B wird die Versteifung wieder aufgehoben.

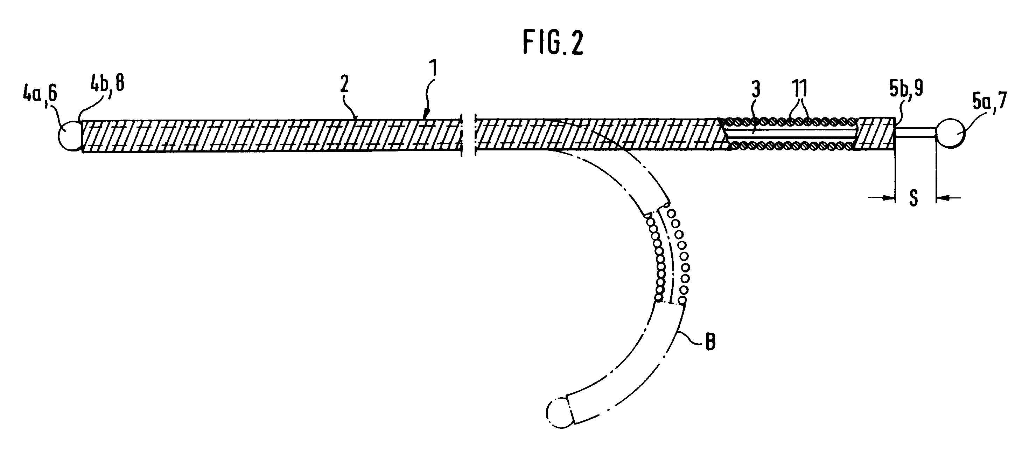

Die Ausgestaltung nach Figur 2 unterscheidet sich von der vorher beschriebenen dadurch, daß die Wicklungen 11 keinen axialen Abstand a voneinander aufweisen, sondern aneinanderliegen. Dabei ist in der geraden Neutralstellung gemäß Figur 2 die Länge der Seele 3 so bemessen ist, daß ein Seelenkopf, hier der hintere Seelenkopf 7, einen Abstand vom zugehörigen Stirnende 9 des Mantels 2 aufweist, der dem Verschiebespiel S entspricht. Da die Seele 3 im Mantel 2 frei versehiebbar ist, können natürlich beide Seelenköpfe 6, 7 einen Abstand von den zugehörigen Stirnenden 8, 9 aufweisen, wobei die Summe dieser beiden Abstände dem Verschiebespiel S entspricht.

Wenn bei der Ausgestaltung gemäß Figur 2 die Sonde 1 gebogen wird, wird in vergleichbarer Weise wie beim vorbeschriebenen Ausführungsbeispiel das Verschiebespiel S aufgehoben, wonach aufgrund dieser oder einer weiteren Biegung B die axiale Spannung zwischen dem Mantel und der Seele und die Versteifung herbeigeführt werden. An der Innenseite der Biegung B bleiben die Wicklungen 11 in gegenseitiger Anlage, während auf der Außenseite der Biegung B die Wicklungen 11 voneinander beabstandet werden, wie es im Bereich der Biegung B in Figur 2 dargestellt ist. Die Aufhebung des Verschiebespiels S ist daran zu erkennen, daß der Seelenkopf 7 am ihm zugewandten Ende des Mantels 2 anliegt. Bereits in dieser Stellung ist eine gewissen Versteifüng vorgegeben. Durch eine weitere Kraftausübung durch Biegen wird die Versteifung vergrößert.

Bei beiden vorbeschriebenen Ausgestaltungen ist die Sonde 1 bezüglich ihrer Enden gleich ausgebildet, d. h. das vordere und das hintere Ende der Sonde 1 sind gleich und sie kann deshalb mit einem wahlweisen Ende in den zu behandelnden Körper eingeführt werden.

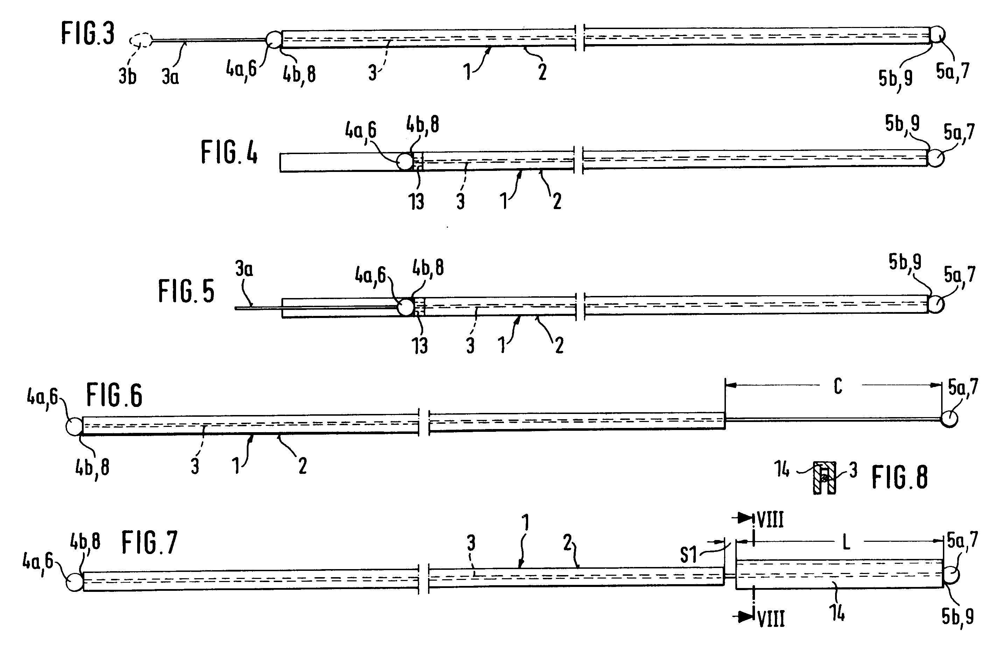

Wie die Figuren 3 bis 5 zeigen, brauchen die Verschiebungs-Begrenzungselementenpaare 4a, 4b, 5a, 5b nicht an den Enden der Sonde 1 angeordnet zu sein. Gemäß Figur 3 erstreckt sich vom vorderen Seelenkopf 6 ein Seelenabschnitt 3a nach vorne, der an seinen freien Ende eine insbesondere rundkopfförmige Verdickung 3b trägt, wie es in Figur 3 andeutungsweise dargestellt ist. Vorzugsweise weist die Verdickung 3b die axiale Querschnittsform eines Kegels auf, dessen gerundete Spitze nach vorne weist, wobei der Kegel an seiner Rückseite ebenfalls gerundet kegelförmig abgeschrägt oder angefast ist. Die Verdickung 3b ist verschweißt, verklebt oder vorzugsweise verlötet. Gemäß Figur 4 ist der Mantel 2 am vorderen oder hinteren Ende über den zugehörigen Seelenkopf 6 hinaus verlängert, wobei das mantelseitige Verschiebungs-Begrenzungselemententeil durch eine Innenschulter 13 im Mantel 2 gebildet wird, die durch einen darin befestigten Ring gebildet sein kann.

Die Figur 5 zeigt eine Kombination der Ausgestaltungen gemäß Figuren 3 und 4, nämlich einen vorderen in den Mantel 2 versenkt an einer Innenschulter 13 anliegenden Seelenkopf 6, von dem sich ebenfalls ein Einführungs-Seelenabschnitt 3a nach vorne erstreckt.

Bei der Ausgestaltung der Sonde 1 gemäß den Figuren 6 bis 8 weisen die Seelenköpfe 6, 7 einen Abstand voneinander auf, der wesentlich größer bemessen ist, als die Länge des Mantels 2, so daß sich am hinteren Ende ein Abstand C zwischen dem Seelenkopf 7 und dem Mantel 2 ergibt, der wesentlich größer ist als das Verschiebespiel S bei den vorbeschriebenen Ausgestaltungen.

Einer solchen Sonde 1 ist ein Abstandhalter 14 zugeordnet, der den Abstand C überbrückt. Der Abstandhalter 14 ist seitlich auf die Seele 3 aufgesteckt, vorzugsweise aufgeklemmt. Es eignet sich eine U-förmige Querschnittsform für den Abstandhalter 14, wobei die Seele 3 zwischen den Schenkeln der U-Form aufgenommen bzw. manuell lösbar geklemmt ist.

Vorzugsweise sind der Sonde 1 mehrere Abstandhalter 14 in unterschiedlichen Längen und bestimmten Längenabstufungen zugeordnet. Hierdurch ist es möglich ein und dieselbe Sonde 1 für verschiedene Körpereingriffe, z. B. in mehr oder weniger gekrümmten oder geschlungenen Körperhöhlen zu benutzen, wobei nach der Einführung der Sonde in einen zu behandelnden Körper ein bezüglich seiner Länge ein zum sich ergebenden Abstand C passender Abstandhalter 14 ausgesucht und aufgesetzt werden kann. Durch die Länge dieses Abstandhalters 14 laßt sich ein gegebenenfalls erwünschtes Verschiebespiel S1 einstellen und somit auch die Stärke der Biegung B vorbestimmen.

Alle vorbeschriebenen Ausgestaltungen eignen sich sowohl für eine solche Sonde 1 als bei der die Wicklungen 11 den Abstand a voneinander aufweisen (Figur 1), als auch für solche Sonde 1, bei der die Wicklungen 11 aneinanderliegen (Figur 2). Im ersten Fall ist es vorteilhaft, einen Abstandhalter 14 mit einer lange L zu wählen, die dem Abstand C entspricht. Im zweiten Fall kann ein Abstandhalter 14 gewählt werden, dessen Länge L ebenfalls dem Abstand C entspricht oder etwas kürzer bemessen ist, wodurch sich das Verschiebespiel S1 als Differenz aus dem Abstand C und der Länge L ergibt.

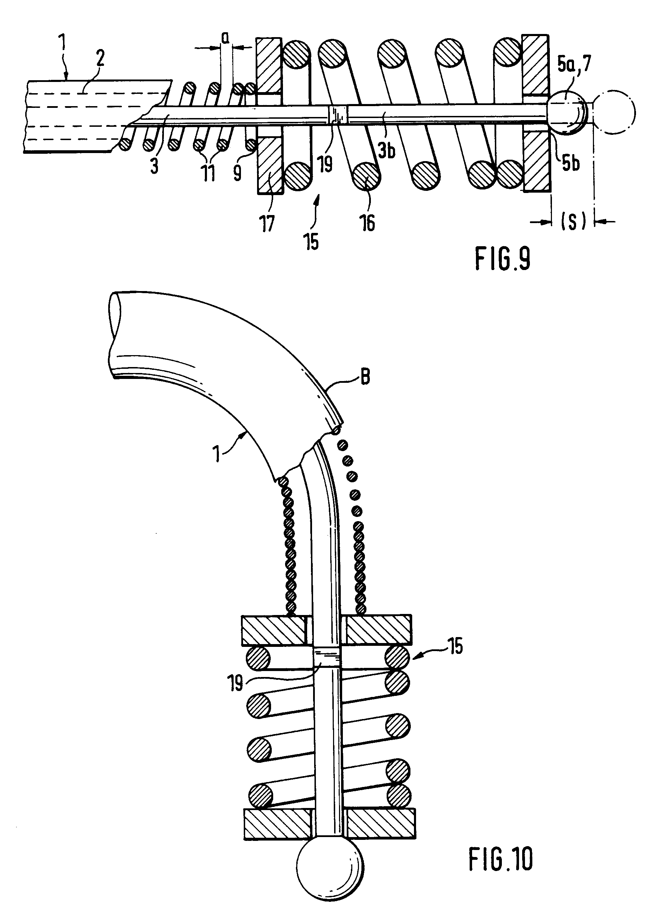

Bei der Benutzung einer erfindungsgemäßen Versteifungssonde 1 besteht die Gefahr, daß durch ein Überspannen der Sonde 1, gegebenenfalls bei mehrmaligem Umwickeln, z. B. um die Hand des Benutzers, das Band oder der Draht der Seele 3 reißt, wodurch die Verbindung der Sonde 1 mit dem vorderen Seelenkopf unterbrochen wird und der Seelenkopf 6 mit der Sonde 1 nicht mehr aus dem Körper herausgezogen werden kann. Um eine solche Überspannung bzw. Überlastung der Seele 3 zu vermeiden, ist der Sonde 1 gemäß der Figuren 9 und 10 eine Zugkraft-Anzeigevorrichtung 15 zugeordnet, die die vorhandenen Zugkraft mißt und mittels eines Anzeigeelements visuell erkennbar macht. Die Anzeigevorrichtung 15 ist koaxial zur Sonde 1 an deren hinterem Ende montiert, d. h. sie ragt von der Sonde 1 nach hinten, und sie besteht aus einer Druckfeder 16, die am rückseitigen Ende des Mantels 2, z. B. mittels eines Zwisehenrings 17, starr gehalten ist, z. B. durch Schweißen, Löten oder Kleben. Die Seele 3 erstreckt sich als vorzugsweise einstückige Verlängerung 3b durch die Druckfeder 16, wobei der hintere Seelenkopf 7 am rückseitigen Ende dieser Verlängerung 3b befestigt ist. Bei einer Ausgestaltung der Sonde 1 gemäß. Figur 1 mit einem Abstand a zwischen den Wicklungen 11 kann der hintere Seelenkopf 7 am hinteren Ende der Druckfeder 16 oder eines an ihr befestigten Endringes 18 angeordnet sein, wie es bereits in vergleichbarer Weise bei ersten Ausführungsbeispiel beschrieben worden ist. Bei einer solchen Ausgestaltung ist das erforderliche Verschiebespiel S durch die Abstände a vorgegeben. Bei einer Ausgestaltung der Sonde 1 mit aneinanderliegenden Wicklungen gemäß Figur 2 ist das Verschiebespiel S zwischen dem hinteren Seelenkopf 7 und dem hinteren Ende der Anzeigevorrichtung 15 vorzusehen. In der in Figur 9 dargestellten Stellung ist die Druckfeder 16 im wesentlichen entspannt. Wegen der auftretenden Spannkräfte ist die Druckfeder 16 im Durchmesser größer und steifer zu bemessen als die Wicklungen 11.

Beim Biegen der Sonde 1 durch den Benutzer im aus der Körperhöhle herausragenden hinteren Bereich gemäß Figur 10 erfolgt - wie bei den vorbeschriebenen Ausführungsbeispielen - die Relativverschiebung zwischen Mantel 2 und Seele 3, wobei der hintere Seelenkopf 7 eingezogen wird, gegen die Druckfeder 16 drückt und diese komprimiert. Hierdurch ist die die Versteifung herbeiführende Zugkraft durch die Druckkraft der Zugfeder 16 bestimmt. Der Weg, den die Seele 3b dabei zurücklegt, ist eine Größe, von der die Zugkraft abhängig ist. Dieser Weg ist somit ein Indikator für die Zugkraft. Deshalb läßt sich sowohl der Weg und/oder die Zugkraft mittels einer Markierung an der Seele 3 und dem Mantel 2 erkennen. Bei der vorliegenden Ausgestaltung ist eine Markierung 19 an der Seele 3b durch einen Strich oder die Kante eines vorzugsweise rotgefärbten Feldes 19a gebildet. Die Sicherheitsgrenze kann z. B. dann erreicht sein, wenn die Markierung 19 mit dem hinteren Ende des Mantels 2 zusammenfällt.

Bei dieser Ausgestaltung ist die anfängliche Druckkraft der Druckfeder 16 so groß zu messen, daß sie als Spannkraft für die Versteifung der Sonde 1 ausreicht.

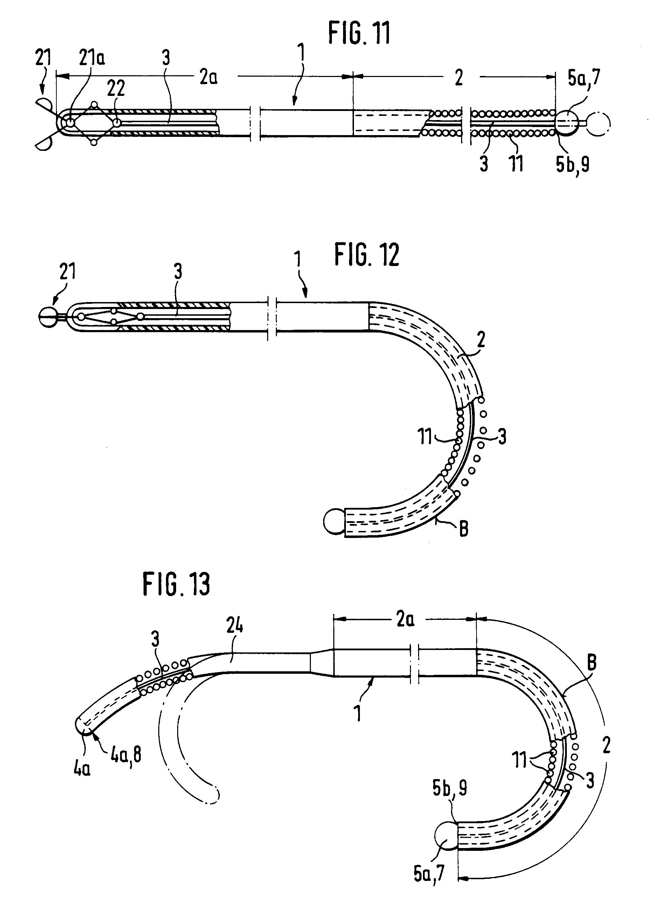

Bei der Ausgestaltung gemäß Figuren 11 und 12 ist der Sonde 1 an ihrem vorderen Ende eine an sich bekannte Biopsiezange 21 in einem am Mantel angeordneten Zangengelenk 21a zugeordnet, deren Zangenteile und Scherenantrieb an sich bekannt ist, so daß auf eine nähere Beschreibung verzichtet werden kann. Die Seele 3 ist mit dem hinteren Scherengelenk 22 verbunden. Da bei dieser Ausgestaltung eine Versteifung der Sonde 1 nicht erforderlich ist, ist nur der hintere, mit 2 bezeichnete Bereich des vorhandenen Mantels durch in Längsrichtung hintereinander angeordnete Elemente, insbesondere Wicklungen 11 gebildet. Der übrige Bereich 2a kann durch ein biegsames Rohr oder einen Schlauch aus Metall oder Kunststoff gebildet sein, der in Längsrichtung möglichst wenig komprimierbar sein soll. Wenn bei dieser Ausgestaltung der Mantel 2 einen Abstand a voneinander aufweisende Wicklungen 11 hat (Figur 1) kann der am hinteren Ende der Seele 3 vorhandene Seelenkopf 7 am hinteren Ende des Mantels 2 angeordnet sein. Wenn die Elemente bzw. Wicklungen 11 des Mantels 2 aneinander liegen, ist zwischen dem Seelenkopf 7 und dem Mantel 2 das Verschiebespiel S vorzusehen. Bei einer Biopsiezange 17 kann gegebenenfalls ein zusätzliches Verschiebespiel vorgesehen sein, um durch manuelles Vorschieben der Seele 3 das Öffnen der Biopsiezange 21 vor der Probeentnahme zu verwirklichen. Bei dieser Ausgestaltung wird die beim Biegen der Sonde 1 in deren hinteren, aus der Körperhöhle herausragendem Bereich stattfindende Relativverschiebung zwischen Mantel 2 und Seele 3, nämlich deren Einschiebung, dazu ausgenutzt, die Biopsiezange 21 zu schließen. Diese Bewegung der Seele 3 wird durch ein oder mehrmaliges Biegen der Sonde 1 problemlos erreicht. Bei dieser Ausgestaltung erweist sich die erfindungsgemäße Lösung als eine Antriebsvorrichtung, die in die Sonde 1 integriert ist.

In mit der vorbeschriebenen vergleichbarer Weise funktioniert auch die Führungssonde nach Figur 13. Bei dieser Ausgestaltung ist die Seele 3 in ansich bekannter Weise am vorderen Ende eines gegebenenfalls im Querschnitt verjüngten Führungsmantels 24 befestigt, der aus elastisch biegsamen Material, wie z. B. Kunststoff oder auch aus Metall in Form von Wicklungen bestehen kann. Der übrige, vom Führungsmantel 24 nach hinten ragenden Mantel 2a besteht ebenfalls aus einem biegsamen Rohr bzw. Schlauch, und er weist in seinem hinteren Bereich einen aus hintereinander liegenden Elementen oder Wicklungen bestehenden Mantel 2 auf. Die vorbeschriebenen, den hinteren Bereich der Sonde 1 gemäß Figur 11 und 12 betreffenden Merkmale gelten auch für die Führungssonde 1 nach Figur 13, so daß eine Wiederholung dieser Ausgestaltungsmerkmale unterbleiben kann. Gezieltes Biegen der Sonde 1 in der hinteren Bereich gemäß Figur 13 laßt sich eine davon abhängige seitliche Ausbiegung des Führungsmantels 24 gezielt herbeiführen. Beim Zurückbiegen der Sonde 1 federt der Führungsmantel 24 selbsttätig in seine in der Regel leicht gekrümmte Ausgangsstellung zurück. Auch bei dieser Ausgestaltung stellt sich die erfindungsgemäße Lösung als integrierte Antriebsvorrichtung dar.

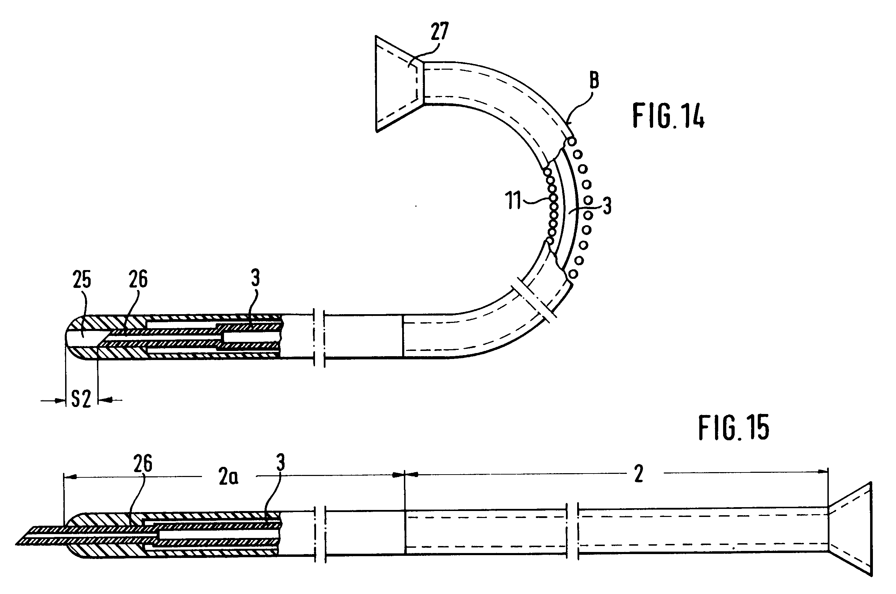

Letzteres gilt auch für das Ausführungsbeispiel gemäß Figur 14 und 15. Bei dieser Lösung wird jedoch durch das Biegen der Sonde 1 aus deren gekrümmten Stellung gemäß Figur 14 in deren gerade Stellung gemäß Figur 15 nicht eine Zugbewegung, sondern eine Schubbewegung der Seele 3 erzeugt. Auch bei dieser Ausgestaltung ist der Mantel 2 nur in seinem hinteren Bereich durch hintereinanderliegende Elemente, insbesondere Wicklungen 11 wie vorbeschrieben gebildet. Der übrige Mantelbereich 2a ist ein elastisch biegsames Rohr bzw. Schlauch aus Metall oder Kunststoff. Auch in diesem Mantel ist ein koaxialer Führungskanal vorhanden, der vorzugsweise im Mantelbereich 2 und im Mantelbereich 2a von gleicher Querschnittsgröße ist und auch mit der gleichen Querschnittsgröße am vorderen Ende des Mantels 2a auslaufen kann. In diesem runden Führungskanal ist eine Seele 3 mit geringem radialem Bewegungsspiel längsverschiebbar aufgenommen, die durch ein biegsames Rohr bzw. eine Kanüle gebildet ist. An ihrem vorderen Ende kann die Seele 3 mit einer Hohlnadel 26 verbunden sein, die im vorderen Endbereich des Mantels 2a längsverschiebbar in einem Führungsloch 25 vorzugsweise Kleineren Durchmessers als der Längskanal gelagert ist. Im hinteren Bereich ist die Seele 3 fest mit einem Spritzen-Anschluß, insbesondere -konus 27 verbunden, der am hinteren Ende des Mantels 2 anliegt und dort befestigt ist. Somit ist das hintere Ende der Seele 3 mit dem hinteren Ende des Mantels 2 fest verbunden und gegenüber diesem nicht verschiebbar. Die lange der Seele 3 und der Hohlnadel 26 ist so bemessen, daß in der geraden, gestreckten Position dieser Sonde 1 die Hohlnadel 26 das vordere Ende des Mantels 2a um das Maß einer Injektions-Einstichtiefe überragt. Durch Abbiegen des hinteren Endbereichs der Sonde im Bereich des Mantels 2 entsprechend den vorbeschriebenen Ausführungsbeispielen wird durch die dabei stattfindende Relativverschiebung zwischen dem Mantel 2, 2a und der Seele 3 die Hohlnadel 26 eingezogen, wie es in Figur 14 dargestellt ist. Ein weiteres Einziehen der Hohlnadel 26 bei einer stärkeren Biegung B ist unschädlich. Bei dieser Ausgestaltung wird die Sonde 1 in gebogenem Zustand (Biegung B) ihres Mantelbereichs 2, in der die Hohlnadel 26 eingezogen ist, in eine Körperhöhle eingeführt. Es ist vorteilhaft, in diesem Zustand einen nach innen gerichteten Abstand zwischen der Spitze der Hohlnadel 26 und dem vorderen Ende des Mantels 2a zu belassen, damit ein dem Verschiebespiel der Ausführungsbeispiele gemäß der Figuren 1 und 2 entsprechendes, hier mit S2 bezeichnetes Verschiebespiel vorhanden ist und die Sonde 1 sich auch gekrümmten Körperhöhlen anzupassen vermag, ohne daß die Hohlnadel 26 aus dem Mantel 2a herauswandert. Die Injizierung erfolgt in der Zielstellung der Sonde 1 in der Körperhöhle im gestreckten Zustand der Sonde gemäß 15. Hierzu wird eine Spritze an den Anschlußkonus 27 angesetzt und durch die hohle Seele 3 injiziert. Es ist vorteilhaft die Sonde 1 im gebogenen Zustand gemäß Figur 14 aus der Körperhöhle wieder herauszuziehen.

Beim Ausführungsbeispiel nach Figuren 14 und 15 umfaßt die Erfindung im Gegensatz zu den Ausgestaltungen gemäß Figuren 11 bis 13 nicht eine Zug-Antriebsvorrichtung sondern eine Schub-Antriebsvorrichtung, die in die Sonde 1 integriert ist.

Bei allen Ausführungsbeispielen ist es vorteilhaft, die Seele 3 aus einem zugfesten bzw. druckfesten Kunststoffmaterial zu fertigen. Hierzu eignet sich Aramid bzw. Kevlar^{R} vorzüglich.

Es ist auch vorteilhaft, die Seele zugelastisch zu gestalten, z. b. aus einem zugelastischen Material wie Kunststoff. Eine solche Ausgestaltung ist insbesondere für Versteifungssonden 1 von Vorteil, um das Zerreißen der Seele 3 oder ein Abreißen von darauf befestigten Bewegungs-Begrenzungsteilen zu verhindern. Die Elastizität sollte dabei so groß sein, daß die von der Seele ausgeübte Spannkraft zur Versteifung der Sonde 1 ausreicht.
Die Ausgestaltung nach Figur 6 bis 8 eignet sich hinsichtlich der Abstandhalteranordnung und -ausbildung auch für die Ausführungsbeispiele nach Figuren 10 bis 13.

Eine Biegung der Sonde 1 insbesondere in ihrem hinteren Endbereich zum Zweck ihrer Versteifung oder Ausführung besonderer Funktionen, zum Beispiel gemäß den Ausführungsbeispielen nach Figuren 11 bis 13, ist mit einem manuellen Bedienungsaufwand verbunden. Auch das Halten der Sonde in der gebogenen Stellung stellt einen Bedienungsaufwand dar und erfordert außerdem Aufmerksamkeit vom Benutzer der Sonde 1. Des weiteren ist sowohl beim Biegen als auch beim Halten der Biegung eine Hand des Benutzers von diesen Tätigkeiten in Anspruch genommen, so daß für andere Tätigkeiten nur die eine Hand des Benutzers zur Verfügung steht. Es ist deshalb vorteilhaft, der Sonde 1 eine Vorrichtung zum Biegen und/oder Halten der Biegung zuzuordnen. Insbesondere beim Halten der Biegung mittels einer geeigneten Vorrichtung stehen dem Benutzer beide Hände für weitere Tätigkeiten zur Verfügung, sofern die die Sonde 1 im gebogenen Zustand haltende Vorrichtung auf einer Unterlage oder an einem Halter abgelegt werden kann.

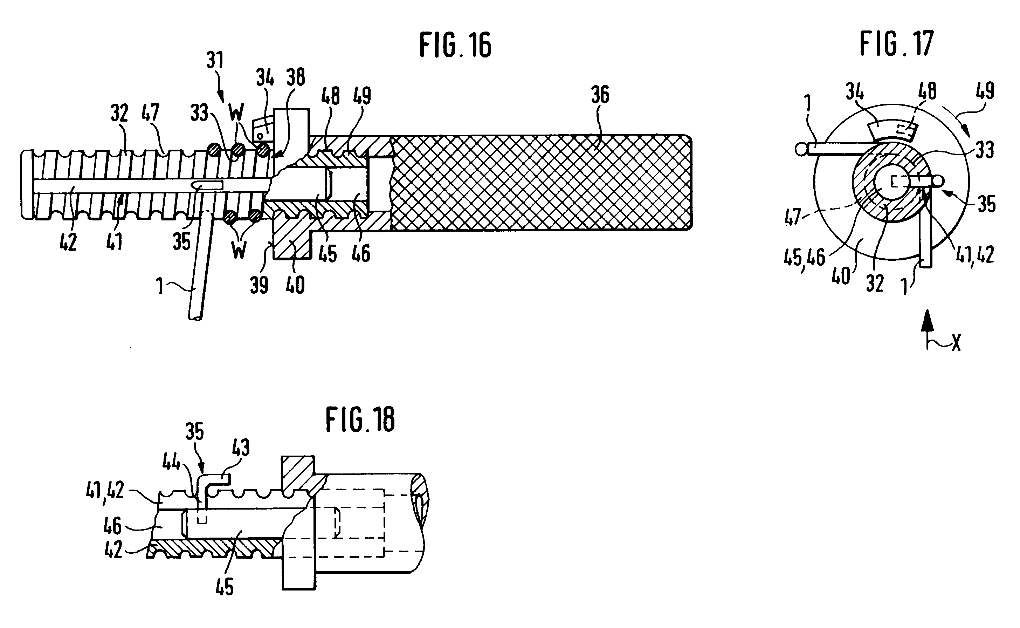

Eine solche Vorrichtung zum Biegen der Sonde und Halten der Biegung ist in den Figuren 16 bis 18 dargestellt und mit 31 bezeichnet. Die Vorrichtung 31 weist einen Biegezapfen 32 mit einer gerundeten Biegefläche 33 auf, um die die Sonde 1 gebogen werden kann. Der Biegezapfen 32 ist vorzugsweise so lang bemessen, daß die Sonde 1 mit mehreren nebeneinanderliegenden Windungen oder Wicklungen W darauf aufgewickelt werden kann. Die Vorrichtung 31 weist zwei Halteelemente 34, 35 auf, die eine Halterung der Sonde 1 im Bereich ihrer Biegung gewährleisten Die Halteelemente 34, 35 sind in der Wickelrichtung bzw. in Umfangsrichtung des Biegezapfens 32 beabstandet, wobei die Biegefläche 33 dazwischen liegt.

Die Vorrichtung 31 erfüllt dann ihren Zweck, wenn sie die gebogene Sonde in ihrem gebogenen Zustand hält. Dies kann durch Übergreifen des gebogenen Abschnitts der Sonde 1 erfolgen. Bei der vorliegenden Ausgestaltung sind zwei, den gebogenen Abschnitt der Sonde 1 in einem in der Wickelrichtung gerichteten Abstand voneinander angeordnete Übergreifelemente vorgesehen, an denen der gebogene Abschnitt der Sonde 1 aufgrund seiner Biegespannung anliegt und dadurch gehalten ist. Es ist auch vorteilhaft, an der Vorrichtung 31 eine Griffeinrichtung vorzusehen, an der sie manuell gehalten werden kann.

Bei der vorliegenden Ausgestaltung weist die Vorrichtung 31 einen länglichen, geraden Biegezapfen 32 auf, der vorzugsweise koaxial oder in Flucht von einem stabförmigen Handgriff 36 insbesondere etwa runden Querschnitts vorspringt. Beide Halteelemente 34, 35 sind Haltebügel in Form von axialen Vorsprüngen, die in einem solchen radialen Abstand vom Wickelzapfen 32 angeordnet sind, daß dazwischen der Wickelabschnitt der Sonde 1 Platz findet. Das zweite Halteelement 35 ist vorzugsweise in der Längsrichtung des Wickelzapfen 32 verstellbar, so daß die Sonde 1 auch in mehreren in Längsrichtung des Wickelzapfen 32 nebeneinander liegenden Wicklungen W gebogen und dieses Halteelement 35 an die sich ergebende axiale Länge der Wicklung angepaßt werden kann.

Bei der vorliegenden Ausgestaltung ist das erste Halteelement 34 an einer nach vorne weisenden Schulterfläche 39 des Handgriffs 36 angeordnet, der hier einen Ringbund 40 aufweisen kann. Das zweite Halteelement 35 ist bezüglich des ersten Halteelements 34 in Umfangs- bzw. Wickelrichtung versetzt angeordnet und in einer axialen Führung 41 wahlweise verschiebbar, die durch eine Längsnut 42 im Wickelzapfen 32 gebilet ist, die sich im wesentlichen von der Schulterfläche 39 bis in den freien Endbereich des Wickelzapfens 32 erstreckt. Das zweite Halteelement 35 ist mit einem axialen Bügelschenkel 43 und einem letzteren tragenden radialen Bügelschenkel 44 winkelförmig geformt. Der radiale Bügelschenkel 44 ist an einem stiftförmigen Gleitstück 45 befestigt, insbesondere in eine radiale Bohrung fest eingesetzt. Das Gleitstück 45 ist in einer koaxialen Längsbohrung 46 im Wickelzapfen 32 mit Bewegungsspiel verschiebbar gelagert, wobei die Längsnut 42 sich in Form eines Schlitzes bis in die Längsbohrung 46 hinein erstreckt und der radiale Bügelschenkel 44 die Längsnut 42 durchfaßt. Der axiale Bügelschenkel 43 ist vorzugsweise nach hinten, das heißt in Richtung auf das erste Halteelement 34 gerichtet.

Bei der vorliegenden Ausgestaltung ist in die Mantelfläche des Wickelzapfens 32 eine Aufnahmenut 47 für die Sonde 1 wendelförmig eingearbeitet, die sich bis zum vorderen Ende des Wickelzapfens erstrecken kann. Hierdurch ist ein geordnetes Aufwickeln möglich, und es bedarf für diesen Vorgang wenig Aufmerksamkeit. Da der gewickelte Abschnitt der Sonde 1 in der Aufnahmenut 47 im Wickelzapfen 32 versenkt angeordnet ist, brauchen die Halteelemente 34, 35 lediglich die zugehörige Wicklung zu übergreifen, das heißt, ein radialer Abstand zur Mantelfläche des Wickelzapfen 32 kann geringer sein oder entfallen, wenn die Querschnittsabmessung der Aufnahmenut 47 dem Durchmesser der Sonde 1 entspricht.

Bei der vorliegenden Ausgestaltung ist die Vorrichtung 31 mit dem Wickelzapfen 32 und dem Handgriff 36 zweiteilig ausgeführt, wobei der Wickelzapfen 32 mit seinem hinteren Ende in ein Aufnahmeloch 48 des vorzugsweise hülsenförmigen Handgriffs 36 fest eingesetzt oder eingeschraubt ist durch einen Eingriff, der zwischen der wendelförmigen Aufnahmenut 47 vorhandenen Nutstege 49 in ein entsprechendes Innengewinde im vorderen Endbereich des Handgriffs 36.

Für einen Wickelvorgang wird das hintere Ende der Sonde 1 in die unter dem ersten Halteelement 34 vorlaufende, die Strecköffnung 38 bildende Aufnahmenut 47 eingesteckt und dann wird die Sonde 1 mit wahlweisen Wicklungen W auf den Wickelzapfen 32 und in der wendelförmigen Aufnahmenut 42 aufgewickelt, bis die Sonde 1 versteift ist oder ihre beabsichtigte Funktion ausgeführt hat. Sofern diese Funktionsstellung beibehalten werden soll, wird das zweite Halteelement 35 axial zu den Wicklungen W hin insoweit verschoben, bis es die vorderste Wicklung W übergreift. Durch die vorhandene Biegespannung wird das Halteelement 35 in seiner Übergreifposition gehalten. In dieser Stellung stehen beide Bedienungshände des Benutzers für andere Aufgaben zur Verfügung.

Im das erste Halteelement 34 bildenden Vorsprung ist ein Sackloch 48 angeordnet, das in der der Biege- bzw. Wickelrichtung (Pfeil 49) offen ist
Die Vorrichtung 31 bzw. der Wickelzapfen 32 und der Handgriff 36 bestehen vorzugsweise mit allen weiteren Einzelteilen insbesondere aus Aluminium.

## Patentansprüche

1. Sonde (1) für medizinische Eingriffe in Körperhöhlen, bestehend aus einem elastisch biegsamen Mantel (2), der durch in Längsrichtung hintereinanderliegende Mantelelemente (11) gebildet ist, und einer Seele (3), wobei
im hinteren, proximalen Bereich der Sonde (1) eine Festlegung (5a, 5b) zwischen dem Mantel (2) und der Seele (3) vorgesehen ist, und wobei in derem vorderen, distalen Bereich eine vordere Festlegung (4a, 4b) zwischen dem Mantel (2) und der Seele (3) vorgesehen ist und ein Verschiebeabstand (S) zwischen Mantel (2) und Seele (3) zugelassen ist,
**dadurch gekennzeichnet,**
daß der Verschiebeabstand ein Verschiebespiel ist, wobei durch Biegen (B) der Sonde (1) in derem hinteren, aus der Körperhöhle herausragenden Bereich eine Relativverschiebung bzw. eine Schub- oder Zugkraft zwischen dem Mantel (2) und der Seele (3) dadurch entsteht, daß die Mantelteile (11) unter Aufhebung des Verschiebespieles (S) an der Innenseite der Biegung (B) aneinander anliegen und an deren Außenseite voneinander beabstandet sind.

2. Sonde nach Anspruch 1,
**dadurch gekennzeichnet,**
daß eine der Festlegungen durch eine starre Fixierung wie eine Verklemmung, Verschweißung, Verlötung oder Verklebung gebildet ist und die andere Festlegung durch ein Längsverschiebungs-Begrenzungselementenpaar (5a, 5b, 4a, 4b) gebildet ist, welches das Verschiebespiel (S) bestimmt.

3. Sonde nach Anspruch 1 oder 2,
**dadurch gekennzeichnet,**
daß der Mantel (2) wenigstens in einem hinteren Bereich oder über seine gesamte Länge aus einem Band oder Draht wendelförmig gewickelt ist, wobei die Wicklungen (11) aneinanderliegen oder wenigstens einen Teil der Wicklungen einen Abstand (a) voneinander aufweisen.

4. Sonde nach Anspruch 2,
**dadurch gekennzeichnet,**
daß das Verschiebungs-Begrenzungselementenpaar (4a, 4b, 5a, 5b) jeweils durch einen Seelenkopf (6, 7) gebildet ist, der mit einer ihm zugewandten Schulter (8, 9, 13) des Mantels (2) zusammenwirkt.

5. Sonde nach Anspruch 4,
**dadurch gekennzeichnet,**
daß die Seelenköpfe (6, 7) mit den Stirnenden (8, 9) des Mantels (2) zusammenwirken.

6. Sonde nach Anspruch 1,
**dadurch gekennzeichnet,**
daß ihre Enden einander gleich ausgestaltet sind.

7. Sonde nach Anspruch 1,
**dadurch gekennzeichnet,**
daß ihr vorzugsweise im hinteren Bereich ein die Relativverschiebung anzeigender Wegmesser und/oder Kraftmesser, insbesondere Zugkraftmesser (15) zugeordnet ist.

8. Sonde nach Anspruch 7,
**dadurch gekennzeichnet,**
daß der Wegmesser und/oder Kraftmesser (15) eine im hinteren Endbereich der Sonde (1) zwischen dem Mantel (2) und dem Verschiebungs-Begrenzungselemententeil (7) der Seele (3) angeordnete Feder, insbesondere Druckfeder (16), vorzugsweise in Form einer Wendelfeder, aufweist.

9. Sonde nach Anspruch 7 oder 8,
**dadurch gekennzeichnet,**
daß am Mantel (2) oder Anbauteilen (15) desselben und an der Seele (3) oder Anbauteilen desselben miteinander korrespondierende Markierungen (19) vorgesehen sind.

10. Sonde nach einem der Ansprüche 7 bis 9,
**dadurch gekennzeichnet,**
daß der Abstand zwischen den an der Seele (3) angeordneten Verschiebungs-Begrenzungselemententeilen (6, 7) gegebenenfalls einschließlich dem Weg- und/oder Kraftmesser (15) größer ist als der Abstand zwischen den dem Mantel (2) zugeordneten Verschiebungs-Begrenzungselemententeilen (4b, 5b) und gegebenenfalls zuzüglich dem Verschiebespiel (S), und daß der Sonde (1) ein Abstandhalter (14) zugeordnet ist, der zwischen dem hinteren, der Seele (3) zugeordneten Verschicbungs-Begrenzungselemententeil (7) und dem hinteren, dem Mantel (2) oder dem Weg- und/oder Kraftmesser (15) zugeordneten Verschiebungs-Begrenzungselemententeil (5b) einsetzbar ist.

11. Sonde nach Anspruch 10,
**dadurch gekennzeichnet,**
daß die Länge (L) des Abstandhalters (14) der Differenz zwischen den der Seele (3) zugeordneten Verschiebungs-Begrenzungselemententeilen (6, 7) und den dem Mantel (2) zugeordneten Verschiebungs-Begrenzungselemententeilen (4b, 5b) entspricht oder in etwa um das Verschiebespiel (S1) kleiner bemessen ist, als diese Differenz.

12. Sonde nach Anspruch 10 oder 11,
**dadurch gekennzeichnet,**
daß der Abstandhalter (14) durch ein U-Profilstück gebildet ist.

13. Verfahren zur Benutzung einer Sonde (1) für medizinische Eingriffe in Körperhöhlen, wobei die Sonde aus einem elastisch biegsamen Mantel (2), der durch in Längsrichtung hintereinanderliegende Mantelelemente (11) gebildet ist, und einer Seele (3) besteht, im hinteren, proximalen Bereich der Sonde (1) eine Festlegung (5a, 5b) zwischen dem Mantel (2) und der Seele (3) vorgesehen ist und daß der Mantel (2) wenigstens in einem hinteren Bereich oder über seine gesamte Länge aus einem Band oder Draht wendelförmig gewickelt ist und wenigstens ein Teil der Wicklungen Abstände (a) voneinander aufweisen, deren Summe ein Verschiebespiel (S) bildet,
**dadurch gekennzeichnet,**
daß durch Biegen (B) der Sonde (1) in deren hinterem, aus der Körperhöhle herausragenden Bereich eine Relativverschiebung bzw. eine Schub- oder Zugspannung zwischen dem Mantel (2) und der Seele (3) dadurch herbeigeführt wird, daß die Mantelelemente (11) unter Aufhebung des Verschiebespieles (S) sich an der Innenseite der Biegung (B) aneinander anlegen und an deren Außenseite voneinander weg bewegen.

14. Verfahren nach Anspruch 13,
**dadurch gekennzeichnet,**
daß die Seele (3) an ihrem vorderen Ende mit einem am vorderen Ende des Mantels (2) portionierten Eingriffswerkzeug, wie einer Injektionsnadel (26), einer Biopsiezange (21) oder einem seitlich ausbiegbaren Führungsende (24) einer sogenannten Führungssonde, verbunden wird.

15. Vorrichtung zur Benutzung einer Sonde nach einem der Ansprüche 1 bis 14,
**gekennzeichnet durch** eine Biegefläche (33) zum Biegen der Sonde (1) über diese Biegefläche (33).

16. Vorrichtung nach Anspruch 15,
**gekennzeichnet durch** eine gerundete, durch die Mantelfläche eines Wickelzapfens (32) gebildete, Biegefläche (33), an der ein Längsabschnitt der Sonde (1) durch ein erstes Halteelement (34) gehalten ist.

17. Vorrichtung nach Anspruch 15 oder 16,
**gekennzeichnet durch** ein zweites Halteelement (35) zum Übergreifen des auf dem Wickelzapfen (32) gewickelten Sondenabschnittes, das vorzugsweise axial verschiebbar am Wickelzapfen (32) gelagert ist.

18. Vorrichtung nach einem der Ansprüche 16 bis 17,
**dadurch gekennzeichnet,**
daß der Wickelzafpen (32) eine wendelförmige Aufnahmenut (47) für die Sonde (1) aufweist.

19. Vorrichtung nach einem der Ansprüche 15 bis 18,
**gekennzeichnet durch** einen Handgriff (36), vorzugsweise in Form eines Stabes, von dem der Wickelzapfen (32) vorspringt, wobei das zweite Halteelement (35) insbesondere in einer Nut (42) des Wickelzapfens (32) axial frei verschiebbar geführt ist.

## Claims

1. Probe (1) for medical procedures in cavities of the body, consisting of an elastically flexible sheath (2), which is formed by sheath elements (11) lying one after another in the longitudinal direction, and a core (3), there being provided in the rearward, proximal region of the probe (1) a fixing (5a, 5b) between the sheath (2) and the core (3), and there being provided in the forward, distal region of the probe a forward fixing (4a, 4b) between the sheath (2) and the core (3) and a displacement spacing (S) between sheath (2) and core (3) being allowed,
characterised in that,
the displacement spacing is a displacement tolerance, whereby by bending (B) of the probe (1) in the rearward region thereof, projecting out of the body cavity, a relative displacement between the sheath (2) and the core (3) or a pushing or pulling force between the sheath (2) and the core (3) arises in that the sheath parts (11) lie against one another on the inner side of the bend (B), the displacement tolerance (S) being taken up, and on the outside of the bend are spaced apart from one another.

2. Probe according to claim 1,
characterised in that,
one of the fixings is formed by a rigid fixing such as by clamping, welding, soldering or adhering and the other fixing is formed by a pair (5a, 5b, 4a, 4b) of longitudinal displacement limiting elements, which pair determines the displacement tolerance (S).

3. Probe according to claim 1 or 2,
characterised in that,
that the sheath (2), at least in a rearward region or over the entire length thereof, is wound of a band or wire in the form of a helix, the windings (11) laying one against the other or at least a part of the windings having a spacing (a) from one another.

4. Probe according to claim 2,
characterised in that,
the pair of longitudinal displacement limiting elements (5a, 5b, 4a, 4b) is in each case formed by means of a core head (6, 7) which cooperates with a facing shoulder (8, 9, 13) of the sheath (2).

5. Probe according to claim 4,
characterised in that,
the core heads (6, 7) cooperate with the ends (8, 9) of the sheath (2).

6. Probe according to claim 1,
characterised in that,
the ends thereof are similarly configured.

7. Probe according to claim 1,
characterised in that,
there is associated therewith, preferably in the rearward region, a travel and/or force detector, in particular a pulling force detector (15), indicating the relative displacement.

8. Probe according to claim 7,
characterised in that,
the travel and/or force detector (15) has a spring, in particular a compression spring (16), preferably in the form of a coil spring, in the rearward end region of the probe (1) between the sheath (2) and the displacement limiting element part (7) of the core (3).

9. Probe according to claim 7 or 8,
characterised in that,
markings (19) corresponding with one another are provided on the sheath (2), or on components (15) attached to the sheath, and on the core (3), or on components attached to the core.

10. Probe according to any of claims 7 to 9,
characterised in that,
the spacing between the displacement limiting element parts (6, 7) arranged on the core (3), if applicable including the travel and/or force detector (15), is greater than the spacing between the displacement limiting element parts (4b, 5b) associated with the sheath (2) and if applicable with the addition of the displacement tolerance (S), and in that a spacer (14) is associated with the probe (1), which spacer can be set between the rearward displacement limiting element part (7) associated with the core (3) and the rearward displacement limiting element part (5b) associated with the sheath (2) or the travel and/or force detector (15).

11. Probe according to claim 10,
characterised in that,
the length (L) of the spacer (14) corresponds to the difference between the displacement limiting element parts (6, 7) associated with the core (3) and the displacement limiting element parts (4b, 5b) associated with the sheath (2), or is less than this difference by approximately the displacement tolerance (S1).

12. Probe according to claim 10 or 11,
characterised in that,
the spacer (14) is formed by a U-profile piece.

13. A method of using a probe (1) for medical procedures in cavities of the body, the probe consisting of an elastically flexible sheath (2), which is formed by sheath elements (11) lying one after another in the longitudinal direction, and a core (3), in the rearward, proximal region of the probe (1) a fixing (5a, 5b) is provided between the sheath (2) and the core (3), and that the sheath (2), at least in a rearward region or over the entire length thereof, is wound of a band or wire in the form of a helix, and at least a part of the windings having spacings (a) from one another, the sum of which spacings provides a displacement tolerance (S),
characterised in that,
by bending (B) of the probe (1) in the rearward region thereof, projecting out of the body cavity, a relative displacement between the sheath (2) and the core (3) or a pushing or pulling force between the sheath (2) and the core (3) is caused in that the sheath elements (11) come to lie against one another on the inner side of the bend (B), the displacement tolerance (S) being taken up, and on the outside of the bend move apart from one another.

14. A method according to claim 13,
characterised in that,
the core (3) is connected at the forward end thereof with a treatment instrument portioned on the forward end of the sheath (2), such as an injection needle (26), biopsy forceps (21) or a laterally deflectable guide end (24) of a so-called guide probe.

15. Device for the use of a probe according to any of
claims 1 to 14,
characterized by a bending surface (33) for bending the probe (1) over this bending surface (33)

16. Device according to claim 15,
characterised by a rounded bending surface (33), formed by the outer surface of a winding pin (32), on which a longitudinal section of the probe (1) is held by a first retaining element (34).

17. Device according to claim 15 or 16,
characterised in that,
a second retaining element (35) is provided for engaging over the probe section wound on the winding pin (32), which element is preferably axially displaceably mounted on the winding pin (32).

18. Device according to any of claims 16 to 17,
characterised in that,
the winding pin (32) has a helical receiving groove (47) for the probe (1).

19. Device according to any of claims 15 to 18,
characterised by a handle (36), preferably in the form of a rod, from which the winding pin (32) projects, the second retaining element (35) being guided freely axially displaceably in particular in a groove (42) of the winding pin (32).

## Revendications

1. Sonde (1) pour interventions médicales dans des cavités corporelles, constituée d'une enveloppe (2) élastiquement souple, qui est formée par des éléments d'enveloppe (11) situés les uns derrière les autres dans la direction longitudinale, et d'une âme (3), dans laquelle, dans la zone proximale arrière de la sonde (1) est prévue une fixation (5a, 5b) entre l'enveloppe (2) et l'âme (3) et dans laquelle, dans la zone distale avant est prévue une fixation avant (4a, 4b) entre l'enveloppe (2) et l'âme (3) et une distance de déplacement (S) est autorisée entre l'enveloppe (2) et l'âme (3), caractérisée en ce que la distance de déplacement est un jeu de déplacement, en ce que par flexion (B) de la sonde (1) dans sa zone arrière, ressortant de la cavité corporelle, un déplacement relatif ou une force de poussée ou une force de traction se produit entre l'enveloppe (2) et l'âme (3), et en ce que les parties d'enveloppe (11) s'appliquent les unes contre les autres sur la face intérieure de la flexion (B), par suppression du jeu de déplacement (S) et sont espacées les unes des autres sur la face extérieure.

2. Sonde selon la revendication 1, caractérisée en ce que l'une des fixations est formée par une fixation rigide telle qu'un serrage, une soudure, une brasure ou un collage et l'autre fixation est formée par une paire d'éléments de délimitation de déplacement longitudinal (5a, 5b, 4a, 4b), qui détermine la jeu de déplacement (S).

3. Sonde selon les revendications 1 ou 2, caractérisée en ce que l'enveloppe (2) est formée au moins sur une zone arrière ou sur toute sa longueur d'un ruban ou d'un fil enroulé an spirale, les enroulements (11) s'appliquant les uns contre les autres où une partie au moins des enroulements présentant une distance (a) les uns des autres.

4. Sonde selon la revendication 2, caractérisée en ce que la paire d'éléments de délimitation de déplacement (4a, 4b, 5a, 5b) est formée par une tête d'âme (6, 7), qui coopère avec un épaulement (8, 9, 13) tourné vers elle de l'enveloppe (2).

5. Sonde selon la revendication 4, caractérisée en ce que las têtes d'âme (6, 7) coopèrent avec les extrémités (8, 9) de l'enveloppe (2).

6. Sonde selon la revendication 1, caractériséé en ce que ses extrémités sont identiques.

7. Sonde selon la revendication 1, caractérisée en ce qu'il lui est associée de préférence dans la zone arrière un dispositif de mesure de distance et/de mesure de force, en particulier un dispositif de mesure de force de traction (15), indiquant le déplacement relatif.

8. Sonde selon la revendication 7, caractérisée en ce que la dispositif de mesure de distance et/ou de mesure de force (15) comporte un ressort, en particulier un ressort de pression (16), de préférence sous la forme d'un ressort spiral, placé dans la zone terminale arrière de la sonde (1), entre l'enveloppe (2) et la partie d'élément de délimitation de déplacement (7) de l'âme (3).

9. Sonde selon les revendications 7 ou 8, caractérisée en ce que sur l'enveloppe (2) ou des accessoires (15) de celle-ci et sur l'âme (3) ou des accessoires de celle-ci sont prévus de marquages (19) correspondant entre eux.

10. Sonde selon l'une des revendications 7 à 9, caractérisée en ce que la distance entre les parties d'éléments de délimitation de déplacement (6, 7), prévue sur l'âme (3), éventuellement y compris la dispositif de mesure de distance et/ou de mesure de force (15), est supérieure à la distance comprise entre les parties d'éléments de délimitation de déplacement (4b, 5b) associées à l'enveloppe (2) et éventuellement augmentées du jeu de déplacement (S), et an ce qu'à la sonde (1) est associé un écarteur (14), qui peut être inséré entre la partie d'élément de délimitation de déplacement (7a) arrière, associé à l'âme (3) et la partie d'éléments de délimitation de déplacement (5b) arrière, associée à l'enveloppe (2) ou au dispositif de mesure de distance et/ou de mesure de force (15).

11. Sonde selon la revendication 10, caractérisée en ce que la longueur (L) de l'écarteur (14) correspond à la différence entre las parties d'éléments de délimitation de déplacement (6, 7) associées à l'âme (3) et les parties d'éléments de délimitation de déplacement (4b, 5b) associées à l'enveloppe (2), ou est plus petite que cette différence, environ du jeu de déplacement (S1).

12. Sonde selon les revendications 10 ou 11, caractérisée en ce que l'écarteur (14) est formé par une pièce profilée an U.

13. Procédé d'utilisation d'une sonde (1) pour interventions médicales dans des cavités corporelles, la sonde étant constituéé d'une enveloppe (2) élastiquement souple, qui est formée par des éléments d'enveloppe (11) situés les uns derrière les autres dans la direction longitudinale, et d'une âme (3), une fixation (5a, 5b) étant prévue dans la zone proximale arrière de la sonde (1), entra l'enveloppe (2) et l'âme (3) et l'enveloppe (2) étant formée au moins dans une zone arrière ou sur toute sa longueur d'un ruban ou d'un fil enroulé en spirale et une partie au moins des enroulements présentant des écartements (a) dont la somme forme un jeu de déplacement (S), caractérisé an ce que par flexion (B) de la sonde (1) dans sa zone arrière, ressortant de la cavité corporelle, il est provoqué un déplacement relatif ou une sollicitation en poussée ou en traction entre l'enveloppe (2) et l'âme (3) par la fait que les éléments d'enveloppe (11) s'appliquent les uns contre les autres, sur la face intérieure de la flexion (8), par suppression du jeu de déplacement (S) et s'écartant les uns des autres sur sa face extérieure.

14. Procédé selon la revendication 13, caractérisé an ce que l'âme (3) est reliée à son extrémité avant avec un outil d'intervention positionné à l'extrémité avant de l'enveloppe (2), tel qu'une seringue (26), une pince de biopsie (21) ou une extrémité de guidage (24), pliable latéralement, d'une sonde dite de guidage.

15. Dispositif pour l'utilisation d'une sonde selon l'une des revendications 1 à 14, caractérisé par une surface de flexion (33) pour plier la sonde (1) sur cette surface de flexion (33).

16. Dispositif selon la revendication 15, caractérisé par une surface de flexion (33) arrondie, formée par la surface d'enveloppe d'une tige d'enroulement (32), sur laquelle une portion longitudinale de la sonde (1) est maintenue par un premier élément de retenue (34).

17. Dispositif selon la revendication 15 ou 16, caractérisé par un deuxième élément de retenue (35) destiné à passer sur la portion de sonde enroulée sur la tige d'enroulement (32), qui est monté de manière à pouvoir de préférence se déplacer axialement sur la tige d'enroulement (32).

18. Dispositif selon l'une des revendications 16 ou 17, caractérisé an ce que la tige d'enroulement (32) présente une rainure de logement (47 en spirale pour la sonde (1).

19. Dispositif selon l'une des revendications 15 à 18, caractérisé par une poignée (36), de préférence sous la forme d'une barre, de laquelle dépasse la tige d'enroulement (32), le deuxième élément de retenue (35) étant guidé de manière à pouvoir coulisser librement axialement, en particulier dans une rainure (42) de la tige d'enroulement (32).
